# EUROPEAN PATENT SPECIFICATION

(11) **EP 1 127 158 B1**
(45) Date of publication and mention of the grant of the patent: **09.01.2008**
(21) Application number: 99952637.9
(22) Date of filing: 29.10.1999
(51) Int. Cl.: C12Q 1/68

(54) **USE OF DNA IDENTIFICATION TECHNIQUES FOR THE DETERMINATION OF GENETIC MATERIAL OF COCOA IN FERMENTED OR ROASTED BEANS AND CHOCOLATE**
VERWENDUNG VON DNA- IDENTIFIZIERUNGSMETHODEN ZUR BESTIMMUNG VON GENETISCHEM COCOAMATERIAL IN FERMENTIERTEN ODER GERÖSTETEN BOHNEN UND SCHOKOLADE
UTILISATION DE TECHNIQUES D'IDENTIFICATION D'ADN POUR LA DETERMINATION DU MATERIEL GENETIQUE DU CACAO A PARTIR DE FEVES ET DE VARIETES DE CHOCOLAT FERMENTEES ET TORREFIEES

(30) Priority: 05.11.1998 EP 98121043
(43) Date of publication of application: 29.08.2001
(73) Proprietor: SOCIETE DES PRODUITS NESTLE S.A., 1800 Vevey (CH)
(72) Inventor: PETIARD, Vincent, F-37100 Tours (FR); CROUZILLAT, Dominique, F-37390 Cerelles (FR)
(74) Representative: Becker Kurig Straus
(86) International application number: PCT/EP1999/008268
(87) International publication number: WO 2000/028078

(56) References cited:
- WO-A-91/19801
- GB-A- 2 283 568
- RUSSELL J ET AL: "Genetic differentiation of Cocoa (Theobroma Cacao L.) populations revealed by RAPD analysis" MOLECULAR ECOLOGY, vol. 2, no. 2, - April 1993 (1993-04) pages 89-97, XP002108807
- LERCETEAU E ET AL: "Evaluation of the extent of genetic variability among Theobroma Cacao accessions using RAPD and RFLP markers" THEOR. APPL. GENET, vol. 95, no. (1-2), 1997, pages 10-19, XP002108808
- WHITKUS R ET AL: "Genetic diversity and relationships of Cacao (Theobroma Cacao L.) in Southern Mexico" THEOR. APPL. GENET., vol. 95, no. 5, April 1998 (1998-04), pages 621-627, XP002108809
- ZIMMERMANN, A, LÜTHY, J AND PAULI, U: "Quantitative and qualitative evaluation of nine different extraction methods for nucleic acids on soya bean food samples" ZEITSCHRIFT FUER LEBENSMITTEL-UNTERSUCHUNG UND -FORSCHUNG A, vol. 207, no. 2, August 1998 (1998-08), pages 81-90,
- LEFRANCOIS P C; HANNI C; LANGE M: "L' ADN anti-fraudes" BIOFUTUR, no. 165, March 1997 (1997-03), pages 27-30, XP004279637
- HELLEBRAND, MAGY, MÖRSEL: "Determination of DNA traces in rapeseed oil" Z LEBENSM UNTERS FORSCH A, vol. 206, 1998, pages 237-242,

## Description

The present invention relates to the use of DNA identification techniques for the determination of genetic material of cocoa in fermented and/or roasted beans and chocolate. In particular, the present invention pertains to the use of techniques selected from the group consisting of PCR, RAPD, RFLP or microsatellite identification and subsequent DNA sequencing, respectively, for the determination of cacao varieties and/or cacao varieties that have been modified by common breeding techniques or that have been modified by genetic engineering in processed beans and chocolate.

Due to the globalisation of the world a huge variety of food products from all over the world have become available in local marketplaces. This also applies to the raw materials or intermediate products used in the various stages of food production.

Apart from new foreign food products entering local markets, novel, modified products may be obtained, that show a variety of different traits. To this end, e.g. tomato paste deriving from genetically engineered tomatoes have been sold in the United Kingdom since 1996 after having been imported from the United States of America.

Due to national regulations some food products and raw materials therefore have to be labelled so as to be able to determine their origin and/or quality level. One of the drawbacks of such labelling procedures resides in that said labels may accidentally or intentionally be exchanged so that the purchaser may not always fully rely on this sort of identification. In addition the enforcement of exclusive proprietary rights on raw materials may require its identification at various processing stages including the end food product on the shelf.

Therefore, there exists a need in the art for a reliable method so as to determine particular raw materials in food products of interest or their origin.

With the progress of DNA analysis techniques efficient methods for the identification of the genetic origin of raw materials/food products have become accessible. However, these techniques may only be applied to products/raw materials the genetic material of which has not been degraded to a substantial degree.

In this respect, R. Greiner at al. report in "Is there any possibility of detecting the use of genetic engineering in processed foods", Z. Ernährungswissenschaft 36 (1977), 155-160, of the option that some food products, such as pizza tomatoes, fried potatoes etc. allow for the detection of their genetic material by means of PCR (Polymerase Chain Reaction; Ehrlich et al., Science 253 (1991), 1643-1651). In the same paper (Greiner et al., supra) it is, however, also mentioned that some processed food products, such as tomato soup, tomato starch, mashed potatoes etc. do not enable such an identification, due to the DNA thereof obviously being degraded to an extensive degree.

For cocoa it was generally accepted that the process steps involved in the preparation of cocoa, such as bean fermentation, drying and roasting, which is generally performed at temperatures of from 105 °C to 150 °C for 20 to 50 minutes, are DNA destructive processing steps such that cocoa DNA cannot be used to identify and control the genetic origin of fermented beans, roasted beans and chocolate.

Consequently, an object of the present invention resides in providing methods for the identification of the genetic material of cocoa in processed cocoa beans and chocolate.
In the studies leading to the present invention it has now surprisingly been found that contrary to the common belief of the state of the art DNA identification techniques are actually suitable to detect genetic material in processed cocoa beans and chocolate and that these techniques may efficiently be used to identify the genetic origin of cocoa.
The above object has therefore been achieved by using DNA identification techniques, in particular the techniques of PCR, RAPD, RFLP or microsatellite and subsequent DNA sequencing, respectively, for the determination of genetic material of cocoa, wherein the material to be determined is fermented beans, roasted beans or chocolate.
By using the above mentioned techniques the identification of cacao (varieties) is now feasible, e.g. from which particular variety the cocoa beans contained in an end product, such as chocolate, have been derived from. This option will also represent an appealing feature so as to determine whether some particular cocoa, such as e.g. cocoa that has been modified or improved by common techniques or that has been subject to genetic engineering or has been utilised in the preparation of the chocolate of interest.

Moreover, the present invention allows for an efficient control of the origin variety of fermented dry beans at the stage of export from/import into a particular country, for an efficient control of the origin of roasted beans in the factory during their processing and for an efficient control of chocolate on the shelf.

The techniques of PCR (Ehrlich et al., supra), RAPD (Random Amplified Polymorphic DNA; Welsh et al., "Fingerprinting genomes using PCR with arbitrary primers", Nucleic acids Res. 18 (1990), 7213-7218), RFLP (restriction fragment length polymorphism; Botstein D. et al., "Construction of a genetic map in man using restriction fragment length polymorphisms" Am. J. Hum. Genet. 32 (1980), 314-331) and microsatellite identification (Tautz, D. et al, "Hypervariability of simple sequences as a general scource for polymorphic DNA markers. Nucl. Acid. Res. 17 (1989), 6463-6471; Weber J. at al., "abundant class of human DNA polymorphisms which can be typed using the polymerase chain reaction", Am J. Hum. Genet. 44 (1989), 388-396) are known and described in the art. The above mentioned documents are incorporated herewith by way of reference.
In a preferred embodiment the DNA to be detected according to the above techniques is a DNA derived from the 5 S gene of cacao or the SSP gene (Seed Storage Protein gene; Spencer E. et Hodge, R., Planta 186 (1992, 567-576) or the chitinase gene.

In a further preferred embodiment the DNA to be detected is derived from mitochondrial and/or chloroplastic DNA. This DNA shows the advantage that it is stably propagated from mother to daughter beans thus enabling to assign the fruits of descendent trees to a particular original tree/variety. This option also applies when the descendents are derived from one female and different male trees.

By applying DNA identification techniques, preferably PCR, RAPD, RFLP or microsatellite identification techniques and subsequent DNA sequencing, respectively, with fermented and/or roasted cocoa beans and/or chocolate it will likewise be possible to identify the presence of a raw material derived from an variety, that has been modified or improved by common breeding techniques or that has been genetically modified, in the end product.

In the figures:
Fig. 1A shows a picture of an agarose gel stained with ethidium bromide, on which cocoa DNA from different scources have been applied.
Fig. 1B shows an autoradiography of a hybridisation experiment, wherein the DNA from the agarose gel of Fig. 1A has been transferred to a nylon membrane and has been hybridized with radioactively labelled cocoa DNA using ³²P as the radioactive isotope.
Fig. 2 shows a picture of an agarose gel stained with ethidium bromide, in which the 5S intergenic spacer on different cocoa samples has been amplified via PCR.
Fig. 3 shows a picture of an agarose gel stained with ethidium bromide, wherein the intron 1 and the exon 2 of the SSP gene has been amplified via PCR on different samples.
Fig. 4 shows RAPD profiles from different cocoa samples.

In the following examples the techniques for the determination of cocoa DNA in fermented and/or roasted beans and chocolate will be described. It is, however, understood that the present invention is not limited to the examples but is rather embraced by the scope of the appended claims.

Unless otherwise indicated the techniques applied are as described in Sambrook J., Fritsch E.F., Maniatis T (1989) Molecular cloning, A Laboratory Manual, second edition, Cold Spring Harbor Laboratory Press (USA).

### Example 1:

### DNA extraction from fermented cocoa beans

400 mg of cocoa embryo axes were collected, ground in liquid nitrogen and homogenised in 10 ml of extraction buffer (Sorbitol 0.35 M, Tris-HCl 0.1 M pH 8, EDTA 5 mM, Sodium bisulphite 5g/l) using an IKA-Ultra-Turrax T25 (Janke & Kunkel). The resulting homogenate is filtrated through Miracloth (Calbiochem) and centrifuged (15 min, 1000 g. 4°C). The pellet is resuspended in 5 ml of extraction buffer and centrifuged (15 min, 1000 g, 4°C). The pellet is resuspended in 0.5 ml of extraction buffer with 0.5 ml of lysis buffer (Tris-HCl 0.2 M pH 8, EDTA 50 mM, NaCl 2 M, CTAB 2%) and 0.1 ml of 5% Sarkosyl and incubated for 60 min at 65°C. The lysate was then extracted with 1 ml of chloroform/isoamyl alcohol (24/1 :v/v). After centrifugation (15 min at 10.000 g, 4°C) the DNA in the aqueous phase was precipitated by adding one volume of isopropanol and standing overnight at -20°C. The DNA was collected by centrifugation, resuspended in 1 ml of TE buffer (Tris-HCl 10 mM pH 8, EDTA 0.1 mM) and purified with RNAse A (Sigma) at a final concentration of 200 µg/ml at 37°C for 30 min, and Proteinase K (Boehringer Mannheim) at a final concentration of 400 µg/ml at 56°C for 1 h. Proteins were removed with phenol/chloroform/isoamyl alcohol (25/24/1:v/v/v) treatment. After centrifugation (15 min, 1000g, 4°C) the DNA from the aqueous phase was precipitated overnight with a mixture of 3 M sodium acetate, pH 5.2, and cold ethanol (0.1/2:v/v; -20°C). The DNA was hooked on a glass rod, washed in 70% ethanol solution and resuspended in 50 µl of TE buffer.

### Example 2:

### DNA extraction from roasted cocoa beans or nibs and chocolate

60 g of dark chocolate (Nestlé, Grands chocolats, Noir (74% cocoa); Auchan, Vendome Noir (52% cocoa), freely available on the market or 25 g of nib (from Nestlé St. Menet, France) were ground in liquid nitrogen and subsequently homogenised in 200 ml of extraction buffer as previously described. The homogenate was filtrated through Miracloth and centrifuged for 15 min at 1000 g at 4°C. The pellet was resuspended in 50 ml of extraction buffer and centrifuged (15 min, 1000 g, 4°C). The pellet was resuspended in 5 ml of extraction buffer with 7 ml of lysis buffer and 2.4 ml of 5% Sarkosyl and incubated for 60 min at 65°C. The resulting lysate was then extracted with 10 ml of chloroform/isoamyl alcohol (24/1:v/v). After centrifugation (15 min, 1000 g, 4°C) the DNA in the aqueous phase was precipitated by adding one volume of isopropanol and standing overnight at -20°C. After two successive centrifugations (15 min at 10.000 g, 4°C) the resulting pellets were pooled and resuspended in 5 ml of TE buffer. Two additional purification steps with phenol/chloroform/isoamyl alcohol (25/24/1:v/v/v) were realised and the DNA from the aqueous phase finally obtained was precipitated overnight by adding 3 M sodium acetate pH 5.2 and cold ethanol (0.1/2:v/v) and standing at -20°C. After centrifugation (15 min, 1000 g, 4°C) the DNA was resuspended in 30 µl of TE buffer. The DNA was purified with RNAse A (final concentration: 200 µg/ml) at 37°C for 10 min.

### Example 3:

### PCR DNA amplification

Two types of PCR amplification were tested, first on repeated DNA sequences from rDNA 5S gene (1000 to 50000 copies per haploid genome) and second on a single gene coding for a cocoa seed storage protein (SSP) with 3 to 5 copies per haploid genome.

### DNA amplification for the intergenic spacer of ribosomal 5S gene:

Purified DNA obtained from cocoa beans, nib or chocolate as illustrated above was diluted in TE buffer with a ratio of 1/500. The DNA amplification is performed with 5 µl of each diluted DNA samples and 45 µl of the PCR buffer containing 200 µM of each dNTP, 25 ng of each primer and 2 U of *Taq* polymerase (Stratagéne) with a final concentration of 1.5 mM magnesium chloride.

The DNA sequences of the two primers used are:
5'-TTTAGTGCTGGTATGATCGC-3' (SEQ. ID. No. I)
5'-TGGGAAGTCCTCGTGTTGCA-3' (SEQ. ID. No. II)

These two primers were designed according to Kolchinsky et al., "Portraying of plant genomes using polymerase chain reaction amplification of ribosomal 5S genes", Genome 34 (1991), 1028-1031. Amplifications were performed in a Bio-Med Thermocycler 60/2 (B. Braun) programmed for a preliminary 2-min denaturation at 94°C and 30 cycles including 1 min at 94°C, 1 min at 55°C and 1 min at 72°C. A final DNA extension cycle was performed at 72°C for 7 min.

DNA amplification for the seed storage protein (SSP) gene:

The DNA amplification procedure was the same as previously described with the proviso that the two DNA primers designed to detect the presence of the SSP gene are as follows:
5'-GGCAATTTACTTCGTGACAAACG-3' (SEQ. ID. No. III)
5'-CTCATATTTGCCAGGAGAATT'AAC-3' (SEQ. ID. No. IV)

### Example 4:

### Random amplified polymorphic DNA (RAPD)

DNA amplification was performed as already described above with the proviso that the primers used are decamers randomly designed from Operon Technologies, Almeda, California.

The Codes for the corresponding primers are as follows:

| | | |
|---|---|---|
| AG15 | 5'-CCCACACGCA-3' | (SEQ ID. NO V) |
| AM10 | 5'-CAGACCGACC-3' | (SEQ ID NO. VI) |

### Z06 (to be found in the PCR kit from Operon)

Amplifications were programmed for 45 cycles including 1 min at 94°C, 1 min at 37°C and 2 min at 72°C. A final DNA extension cycle was performed at 72°C for 7 min according to Williams et al., "DNA polymorphisms amplified by arbitrary primers are useful as genetic markers", Nucl. Acids Res. 18 (1990), 6531-6535.

### DNA electrophoresis and hybridisation:

The different DNA fragments were separated on a 1.4 % agarose gel followed by staining with ethidium bromide. Southern blots were made according Southern (Sambrook, supra) using alkaline capillary transfer to nylon membranes (Appligène). The DNA probes were labelled using ³²P dCTP with the Megaprime kit (Amersham) and were hybridised on the membranes overnight at 65°C in hybridisation buffer (5% w/v SDS, 5 X SSC, 5 X Denhart's solution, 40µg/ml of heterologous DNA). Post-hybridisation treatments consisted of three high- stringency washes (2 X SSC, 0.1% SDS; 1 X SSC, 0.1% SDS; 0.2 X SSC, 0.1% SDS) each at 65°C for 30 min.

### Example 5:

### DNA detection from cocoa bean to chocolate

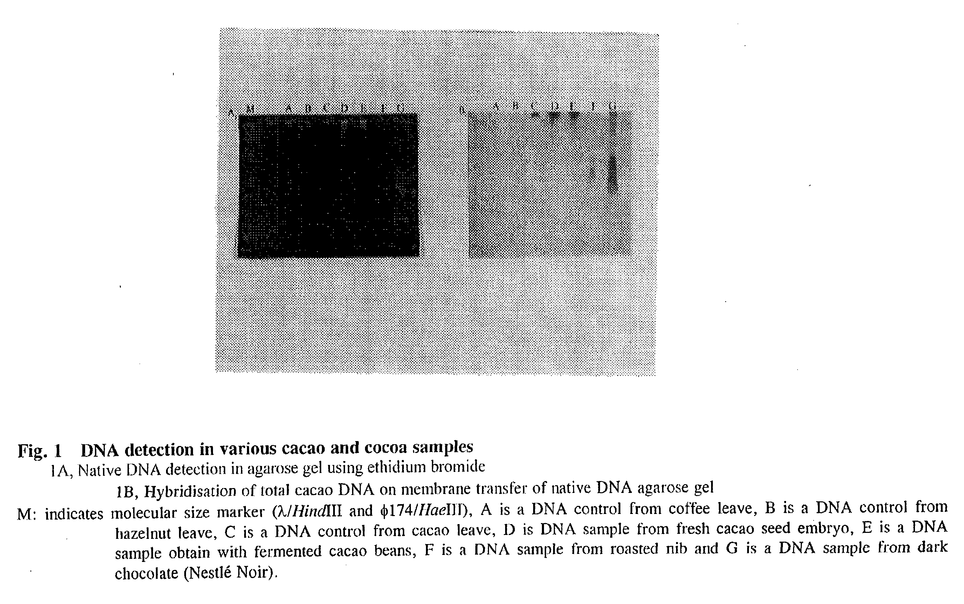

The protocol used allowed the detection of DNA of high molecular weight in fermented cocoa beans and chocolate. As negative control coffee and hazelnut DNA were used. To ensure that the DNA extracted from nib and chocolate was derived from cocoa the DNA transferred to the nylon membrane has been hybridized with radioactively labelled cocoa total DNA purified from cocoa tree leaves. The autoradiography (Figure 1B) indicates positive homology for the cocoa samples from leaves to chocolate which demonstrates that the DNA purified from these samples was originated from cocoa.

### PCR DNA amplification from cocoa samples:

In order to test the possibility to detect specific DNA sequences from the different samples specific DNA primers were used to amplify first the intergenic spacer from the 5S ribosomal gene tandemly repeated and second a part of the seed storage protein gene (SSP).

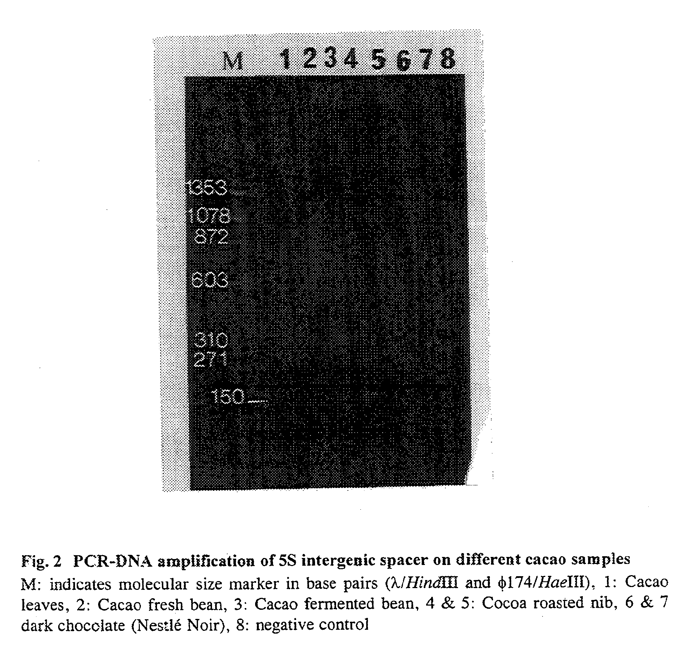

The 5S intergenic spacer amplifications were successful on all the DNA samples tested from leaves to chocolate (Figure 2). These PCR DNA amplifications were detected as a faint DNA smear indicating a substantial degradation of the DNA during the processing steps of the cocoa beans with discrete bands to be detected from approximately 160 bp to up than more 1000 bp for the leaf and fresh bean samples. These multiple DNA bands amplification were the result of the tandem repetition organisation of the target 5S ribosomal gene.

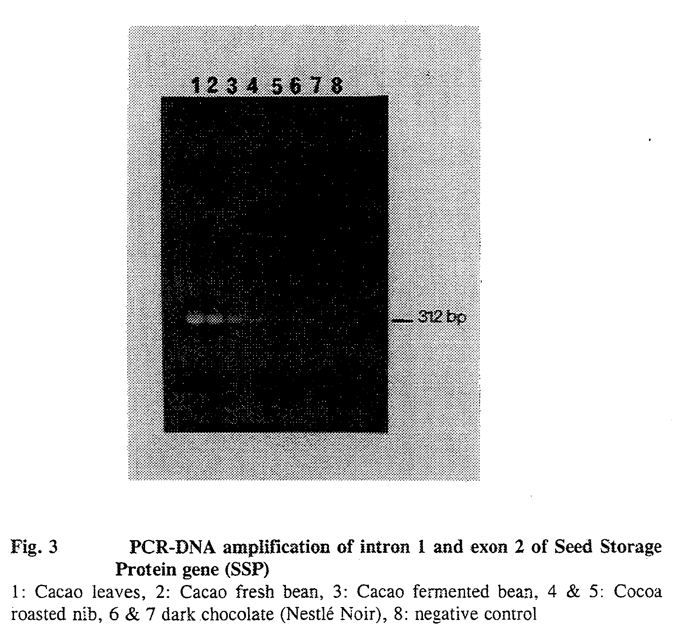

The part of the SSP gene amplified corresponds to the intron 1 and exon 2 thereof and yields a PCR product of 312 bp in length which may be detected in all the samples tested (Figure 3). This result allows to consider that it is possible to detect a single DNA gene copy from leaf to chocolate since SSP gene represents a low gene copy number in *Theobroma cocoa* haploid genome (3 to 5 copies).

DNA polymorphism was detected for the PCR DNA amplification from part of SSP gene.

### RAPD tests on cocoa samples:

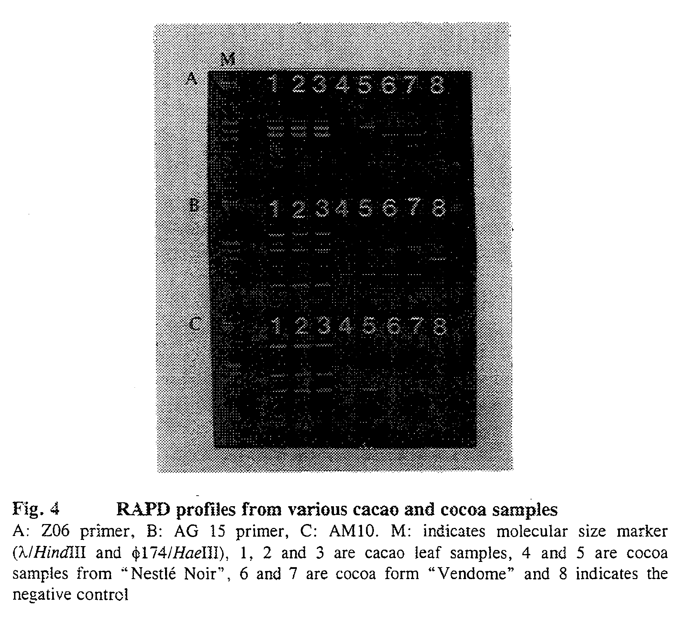

The goal of this experiment was to demonstrate that it could be possible to get RAPD fingerprint from processed cocoa samples to determine the genetic origin(s) of the raw material used for a chocolate. Three primers were selected from Operon kits and the results are illustrated by the figure 4. DNA amplifications were obtained in two different chocolate blends (Nestlé, Grands chocolats, Noir (74% cocoa); Auchan, Vendome Noir (52% cocoa)) showing that it is indeed possible to relate the end product to particular raw materials.

### Example 6:

### DNA sequencing from cocoa bean to chocolate

In order to genetically characterise different cocoa samples from beans to chocolate a PCR amplification of two cocoa genes (seed storage protein gene (SSP) and chitinase gene) was followed by a sequencing to detect DNA polymorphism. Two cocoa trees (Laranja and EET 95) were used to get the different cocoa bean samples and chocolate. The results indicate that the two genotypes used can be assessed by specific DNA sequence of SSP and chitinase genes.

### PCR DNA amplification from cocoa samples:

To amplify specifically the two gene fragments from the different cocoa samples specific DNA primers were used.

The part of the SSP gene amplified corresponds to the introns 1, 2, 3 and exons 2, 3 and yields a PCR product of 584 bp-in length. Purified DNA obtained from cocoa beans, nib or chocolate as shown in example 2 was diluted in TE buffer with a ratio of 1/500. The DNA amplification was performed with 5 µl of diluted DNA samples and 45 µl of the PCR buffer containing 200 µM of each dNTP, 25 ng of each primer and 2U of Taq polymerase (Stratagéne) with a final concentration of 1.5 mM magnesium chloride.
The DNA sequences of the two primers used are:
5'-GGCAATTTACTTCGTGACAAACG-3' (SEQ. ID. No. III)
5'-CCTCCAGCTTCTCTCTTTGTGT-3' (SEQ. ID. No. VII)

Amplifications were performed in a Braun 60/2 thermocycler programmed for 30 cycles including 1 min at 94°C, 1 min at 55°C and 1 min at 72°C. A final extension step was performed at 72°C for 7 min.

A fragment of the chitinase gene was amplified using genomic clone sequence from accession code U30324 of the GCG (Genetics Computer Group, USA). The DNA amplification protocol is the same as described above except that 50 ng of the two DNA primers are used. The length of the PCR amplification is 1064 bp.
The DNA sequences of the two primers used are:
5'-GCTGAGCAGTGTGGACGGC-3' (SEQ. ID. No. VIII)
5'-CCTCTGGTTGTAGCAGTCGA-3' (SEQ. ID. No. IX)

Sequencing of SSP and chitinase gene fragments amplified from fermented beans or chocolate:

Two independent PCR amplifications were carried out for each gene (SSP and chitinase) and for each variety (Laranja and EET 95). 10 ng of DNA from each PCR amplification were cloned in pGEM T easy vector according to Promega instructions. JM109 E. coli competent cells were transformed by recombinant plasmid DNA and grown on LB plates containing ampicillin (100 mg/l), X GAL (40 mg/l) and IPTG (20 mg/l). Five clones, randomly selected, containing SSP or chitinase gene fragments were sequenced.

DNA identification of genetic material from sequences with SSP gene:

The DNA sequences obtained from the SSP gene allows the genetic differentiation of the different cocoa samples tested originated from Laranja and EET 95 cocoas due to two mutations in position 67 (G/A) and 475 (C/G) as described below:
SSP DNA sequence (583 bp) from Laranja cocoa samples:
SSP DNA sequence (583 bp) from EET 95 cocoa samples:
DNA identification of genetic material from sequences with chitinase gene:

The DNA sequences from the chitinase gene discriminate also the different cocoa samples tested originated from Laranja and EET 95 cocoas due to four mutations in position 266 (G/A), 425 (A nucleotide addition/deletion), 615 (A/G) and 865 (A/G) as described below:
Chitinase DNA sequence (1062 bp) from Laranja cocoa samples:
SSP DNA sequence (1063 bp) from EET 95 cocoa samples:

### SEQUENCE LISTING

<110> SOCIETÉ DES PRODUITS NESTLÉ
<120> IDENTIFICATION OF CACAO
<130> Identification of cacao
<140>
   <141>
<150> 98121043.8<151> 1998-11-05
<160> 13
<170> Patent In Ver. 2.1
<210> 1
   <211> 20
   <212> DNA
   <213> cacao
<400> 1
   tttagtgctg gtatgatcgc 20
<210> 2
   <211> 20
   <212> DNA
   <213> cacao
<400> 2
   tgggaagtcc tcgtgttgca 20
<210> 3
   <211> 23
   <212> DNA
   <213> cacao
<400> 3
   ggcaatttac ttcgtgacaa acg 23
<210> 4
   <211> 24
   <212> DNA
   <213> cacao
<400> 4
   ctcatatttg ccaggagaat taac 24
<210> 5
   <211> 10
   <212> DNA
   <213> cacao
<400> 5
   cccacacgca 10
<210> 6
   <211> 10
   <212> DNA
   <213> cacao
<400> 6
   cagaccgacc 10
<210> 7
   <211> 22
   <212> DNA
   <213> cacao
<400> 7
   cctccagctt ctctctttgt gt 22
<210> 8
   <211> 19
   <212> DNA
   <213> cacao
<400> 8
   gctgagcagt gtggacggc 19
<210> 9
   <211> 20
   <212> DNA
   <213> cacao
<400> 9
   cctctggttg tagcagtcga 20
<210> 10
   <211> 583
   <212> DNA
   <213> cacao
<400> 10
   cctccagctt ctctctttgt gtctaacaaa caagataaaa atgaataaat aaataaataa 60
   gtaaaagaca agagaaagta aaaacaaaaa attgattcat agctagtcaa agaaccatat 120
   acattgaaga cggtctcaag aacttcatag ctgaaggctc cgtaatatga ttcaggttta 180
   ttatttccag cggggaagaa taactgcagc aattataagt acagggtcaa tagactaacc 240
   aagacatcaa ggttatgtag aaacttctaa taaataaatg ttaaagtaga aaacctcata 300
   tttgccagga gaattaacag gcagggcgag cacagctatg gttagcttct cttggttgtc 360
   ttggctaacc acgtaaacag tgcttcctgc aggaacgctg actactgttc cacgctgtac 420
   attataggac tctttgtttt catgagtcac aaacgtaatt gtcccctttc ctgacacaga 480
   aataatttac tatgttttca atcaatggtg atttggtgat aaaagccgca aaattttgtt 540
   cgaaagggaa gagaatttac cgtttgtcac gaagtaaatt gcc                   583
<210> 11
   <211> 583
   <212> DNA
   <213> cacao
<400> 11
   cctccagctt ctctctttgt gtctaacaaa caagataaaa atgaataaat aaataaataa 60
   gtaaaaaaca agagaaagta aaaacaaaaa attgattcat agctagtcaa agaaccatat 120
   acattgaaga cggtctcaag aacttcatag ctgaaggctc cgtaatatga ttcaggttta 180
   ttatttccag cggggaagaa taactgcagc aattataagt acagggtcaa tagactaacc 240
   aagacatcaa ggttatgtag aaacttctaa taaataaatg ttaaagtaga aaacctcata 300
   tttgccagga gaattaacag gcagggcgag cacagctatg gttagcttct cttggttgtc 360
   ttggctaacc acgtaaacag tgcttcctgc aggaacgctg actactgttc cacgctgtac 420
   attataggac tctttgtttt catgagtcac aaacgtaatt gtcccctttc ctgagacaga 480
   aataatttac tatgttttca atcaatggtg atttggtgat aaaagccgca aaattttgtt 540
   cgaaagggaa gagaatttac cgtttgtcac gaagtaaatt gcc                   583
<210> 12
   <211> 1062
   <212> DNA
   <213> cacao
<400> 12
   gctgagcagt gtggacggca agctggtggt gccctgtgcc ctggaggcct atgttgtagc 60
   caatttggtg ggtgtggcaa cactgatgac tactgcaaaa gggaaaatgg ttgccagagt 120
   cagtgcagcg gaagcggagg tgatactggt ggacttgata gtctgataac aagagaaagg 180
   tttgatcaga tgcttttgca tagaaatgat ggtggttgtc ctgctcgtgg cttctatacc 240
   tatgatgctt tcatagctgc tgcgaggtct ttccctgcct tcgctacaac cggtgatgat 300
   gccactcgca agagggaagt tgctgctttc ttggcccaaa cttctcacga aactactggt 360
   tagtccactt cgaaagttaa tcacaaagtt caccatgttt tgaacatgac ttcatcggtt 420
   tgagattaat ttgatgatgc cgtaggtgga gcaggatggg ctgcacccga tggtccatat 480
   acgtggggat actgctacaa tagggaatta aaccccgctg attactgcca gtgggatcca 540
   aactaccctt gcgctcctgg taagcaatat tttggccggg gtccaatgca acttacttgg 600
   taagcctttc accatttgct aatttctttt cttgaaatgt atttatggta aggcaaaatt 660
   gttttgttga catgggaata atcacttaac ttttgatata tcaggaacta caactatggg 720
   cagtgtggaa gagccattgg ggtggaccta ttaaacaacc cagacctgct agcaactgat 780
   cctacaattt ctttcaagtc agcgttctgg ttctggatga ctccacaatc accaaagcct 840
   tcttgccacg atgtgatcat tggagcgtgg tcaccctccg gtagcgacca ggcggcaggc 900
   cgggttccag ggtttggttt gatcacaaat attatcaatg gcggccttga atgtggtcaa 960
   ggttggaatg caaaggtaga ggaccgcatt gggttctata agaggtattg tgacacactt 1020
   ggagttggct atggtaacaa tctcgactgc tacaaccaga gg                    1062
<210> 13
   <211> 1063
   <212> DNA
   <213> cacao
<400> 13
   gctgagcagt gtggacggca agctggtggt gccctgtgcc ctggaggcct atgttgtagc 60
   caatttggtt ggtgtggcaa cactgatgac tactgcaaaa aggaaaatgg ttgccagagt 120
   cagtgcagcg gaagcggagg tgatactggt ggacttgata gtctgataac aagagaaagg 180
   tttgatcaga tgcttttgca tagaaatgat ggtggttgtc ctgctcgtgg cttctatacc 240
   tatgatgctt tcatagctgc tgcgaagtct ttccctgcct tcgctacaac cggtgatgat 300
   gccactcgca agagggaagt tgctgctttc ttggcccaaa cttctcacga aactactggt 360
   tagtccactt cgaaagttaa tcacaaagtt caccatgttt tgaacatgac ttcatcggtt 420
   tgagaattaa tttgatgatg ccgtaggtgg agcaggatgg gctgcacccg atggtccata 480
   tacgtgggga tactgctaca atagggaatt aaaccccgct gattactgcc agtgggatcc 540
   aaactaccct tgcgctcctg gtaagcaata ttttggccgg ggtccaatgc aacttacttg 600
   gtaagccttt caccgtttgc taatttcttt tcttgaaatg tatttatggt aaggcaaaat 660
   tgttttgttg acatgggaat aatcacttaa cttttgatat atcaggaact acaactatgg 720
   gcagtgtgga agagccattg gggtggacct attaaacaac ccagacctgc tagcaactga 780
   tcctacaatt tctttcaagt cagcgttctg gttctggatg actccacaat caccaaagcc 840
   ttcttgccac gatgtgatca ttggggcgtg gtcaccctcc ggtagcgacc aggcggcagg 900
   ccgggttcca gggtttggtt tgatcacaaa tattatcaat ggcggccttg aatgtggtca 960
   aggttggaat gcaaaggtag aggaccgcat tgggttctat aagaggtatt gtgacacact 1020
   tggagttggc tatggtaaca atctcgactg ctacaaccag agg                   1063

## Claims

1. Process for obtaining DNA from fermented/roasted cocoa beans comprising the steps of:
providing cocoa material;
grounding the material in liquid nitrogen;
homogenizing, filtrating and centrifuging said material to obtain a pellet;
resuspending the pellet and lysing;
extracting the lysate; and
precipitating DNA.

2. Use of the DNA obtained by the process according to claim 1 for a DNA detection technique for the determination of genetic material of cocoa in fermented and/or roasted beans and/or chocolate.

3. The use according to claim 2, wherein the DNA detection technique is selected from the group consisting of PCR, RAPD, RFLP or microsatellite identification.

4. The use according to claim 2 wherein the DNA analysis technique is sequencing of PCR amplified genes.

5. The use according to any of the claims 2 or 3, wherein the DNA to be detected is derived from the 5 S gene of cocoa.

6. The use according to claim 2, wherein the DNA to be detected is derived from the SSP gene of cocoa.

7. The use according to claim 2 wherein the DNA to be detected is derived from the chitinase gene of cocoa.

8. The use according to claim 2, wherein the DNA to be detected is derived from mitochondrial and/or chloroplastic DNA of cocoa.

9. The use according to any of the claims 2-8, wherein the cocoa from which the fermented and/or roasted beans and the chocolate are derived, is a variety, that has been modified by common breeding techniques or that has been modified by genetic engineering.

## Patentansprüche

1. Verfahren zum Erhalten von DNA von fermentierten/gerösteten Kakaobohnen, umfassend die Schritte:
Bereitstellen von Kakaomaterial,
Absetzten lassen des Materials in flüssigem Stickstoff,
Homogenisieren, Filtrieren und Zentrifugieren des Materials, um ein Pellet zu erhalten,
Resuspendieren des Pellets und Lysieren,
Extrahieren des Lysats, und
Präzipitieren von DNA.

2. Verwendung der durch das Verfahren nach Anspruch 1 erhaltenen DNA für ein DNA-Nachweisverfahren, um genetisches Material von Kakao in fermentierten und/oder gerösteten Bohnen und/oder Schokolade zu bestimmen.

3. Verwendung nach Anspruch 2, wobei das DNA-Nachweisverfahren ausgewählt ist aus der Gruppe bestehend aus PCR, RAPD, RFLP oder Identifikation von Mikrosatelliten.

4. Verwendung nach Anspruch 2, wobei das DNA-Analyseverfahren das Sequenzieren von durch PCR amplifizierten Genen darstellt.

5. Verwendung nach einem der Ansprüche 2 oder 3, wobei die nachzuweisende DNA von dem 5S Gen von Kakao abgeleitet ist.

6. Verwendung nach Anspruch 2, wobei die nachzuweisende DNA von dem SSP Gen von Kakao abgeleitet ist.

7. Verwendung nach Anspruch 2, wobei die nachzuweisende DNA von dem Chitinase-Gen von Kakao abgeleitet ist.

8. Verwendung nach Anspruch 2, wobei die nachzuweisende DNA von der DNA der Mitochondrien und/oder Chloroplasten von Kakao abgeleitet ist.

9. Verwendung nach einem der Ansprüche 2-8, wobei der Kakao, von dem die fermentierten und/oder gerösteten Bohnen und die Schokolade stammen, eine Varietät darstellt, die durch allgemeine Zuchtverfahren oder durch Gentechnik modifiziert wurde.

## Revendications

1. Procédé pour obtenir de l'ADN à partir de fèves de cacao fermentées/torréfiées comprenant les étapes de :
fournir du matériel de cacao;
écraser le matériel dans de l'azote liquide;
homogénéiser, filtrer et centrifuger ledit matériel pour obtenir une boulette ;
remettre la boulette en suspension et lyser ;
extraire le lysat ; et
précipiter l'ADN.

2. Utilisation de l'ADN obtenu par le procédé selon la revendication 1 pour une technique de détection d'ADN pour la détermination de matériel génétique de cacao dans des fèves fermentées et/ou torréfiées et/ou du chocolat.

3. L'utilisation selon la revendication 2 dans laquelle la technique de détection d'ADN est sélectionnée à partir du groupe consistant en PCR, RAPD, RFLP ou identification par microsatellites.

4. L'utilisation selon la revendication 2 dans laquelle la technique de détection d'ADN est le séquençage de gènes amplifiés par PCR.

5. L'utilisation selon l'une quelconque des revendication 2 ou 3 dans laquelle l'ADN à détecter est dérivé du gène 5 S de cacao.

6. L'utilisation selon la revendication 2 dans laquelle l'ADN à détecter est dérivé du gène SSP de cacao.

7. L'utilisation selon la revendication 2 dans laquelle l'ADN à détecter est dérivé du gène de la chitinase de cacao.

8. L'utilisation selon la revendication 2 dans laquelle l'ADN à détecter est dérivé de l'ADN mitochondrial et/ou chloroplastique de cacao.

9. L'utilisation selon l'une quelconque des revendications 2 à 8 dans laquelle le cacao dont les fèves fermentées et/ou torréfiées et le chocolat sont dérivés, est une variété qui a été modifiée par des techniques de reproduction usuelles ou qui a été modifié par génie génétique.
